# EUROPEAN PATENT SPECIFICATION

(11) **EP 3 484 439 B1**
(45) Date of publication and mention of the grant of the patent: **27.07.2022**
(21) Application number: 17730156.1
(22) Date of filing: 14.06.2017
(51) Int. Cl.: A61K 8/37, A61K 8/67, A61Q 15/00, A61K 8/92

(54) **DEODORANT PRODUCTS**
DEODORANTPRODUKTE
DÉODORANTS.

(30) Priority: 14.07.2016 IN 201621024127
(43) Date of publication of application: 22.05.2019
(73) Proprietor: Unilever IP Holdings B.V., 3013 AL Rotterdam (NL); Unilever Global IP Limited, Wirral, Merseyside CH62 4ZD (GB)
(72) Inventor: JAMES, Alexander, Gordon, Bedford Bedfordshire MK44 1LQ (GB); MAJUMDAR, Amitabha, Whitefield Bangalore 560 066 (IN); MCWALTER, Joy, Rachel, Leeds Yorkshire LS14 2AR (GB)
(74) Representative: Whaley, Christopher
(86) International application number: PCT/EP2017/064592
(87) International publication number: WO 2018/010906

(56) References cited:
- WO-A1-02/069923
- DE-A1- 2 160 408
- US-A1- 2003 049 290
- US-A1- 2007 212 324
- DATABASE GNPD [Online] MINTEL; October 2013 (2013-10), Anonymous: "Whitening Roll-on", XP002773496, Database accession no. 2196247
- DATABASE GNPD [Online] MINTEL; October 2014 (2014-10), "Problematic armpit cooling deo mesh stick", XP002773497, Database accession no. 2713215
- DATABASE GNPD [Online] MINTEL; July 2015 (2015-07), Anonymous: "24h Antiperspirant cream dedorant", XP002773498, Database accession no. 3286701

## Description

### Field of Invention

This invention relates to the field of deodorant compositions. In addition, this invention is concerned with achieving a deodorancy benefit upon the surface of the human body. The compositions and methods involved utilise anti-microbial deodorant actives. The compositions and methods of the invention are generally of greatest benefit when used on the most malodorous areas of the body, for example the underarm areas or feet.

### Background

Most anti-microbial and deodorant compositions reduce the number of viable microorganisms on the surface of the skin. It is well known that sweat is usually odourless until the skin microflora have degraded it. Typical deodorants include ethanol and triclosan (2,4,4'-trichloro,2'-hydroxy-diphenyl ether) which is a well known anti-microbial agent. However, the deodorising effect obtained with such deodorants wears off with the passage of time and the microbiota progressively recover their numbers.

There is, therefore, a continuing requirement for effective and long lasting deodorant compositions on the market (particularly in the most malodorous areas, eg. the axillae).

DE 21 60 408 A1 (KOEHLER VALENTIN; KOEHLER JULIAN; 8702 GERBRUNN) discloses a deodorant composition comprising nicotinamide and glycerol.

WO 02/069923 (P&G, 2002) discloses anhydrous antiperspirant and deodorant compositions comprising a solid, water-soluble skin active agent (e.g. niacinamide) and glycerol.

EP 1842522 ((Otsuka Pharmaceutical Co., 2015) discloses solid oil-in-water emulsions comprising a triglyceride oil and glycerol, with vitamins as an additional, optional component.

### Summary of the Invention

According to a first aspect of the present disclosure there is provided a deodorant composition comprising water, niacinamide at from 0.5 to 10% by weight, glycerol and triglyceride oil, wherein the total amount of glycerol and triglyceride is greater than the amount of niacinamide present.

We have surprisingly discovered that by topical application of said composition a significant deodorancy benefit can be delivered to the human body.

In a second aspect of the present disclosure there is provided a non-therapeutic method of reducing body odour comprising the topical application of a composition according to the first aspect of the invention to the human body.

Without wishing to be bound by theory, it is believed that the deodorancy benefit delivered by the present composition is obtained primarily by an anti-microbial action upon the skin microbiota. In particular, it is hypothesised that bacteria responsible for the conversion of secretions from the eccrine and/or apocrine glands into malodorous materials are reduced by use of the present composition. Further, it is believed that the glycerol and triglyceride employed may enhance the effectiveness of the niacinamide, which is the primary antimicrobial deodorant active employed.

### Detailed Description

The present invention is described in the present claims and relates to a non-therapeutic method of reducing malodour on the surface of the human body, comprising the topical application of a deodorant composition comprising a) water, b) niacinamide at from 0.5 to 10% by weight, c) glycerol and d) unsaturated triglyceride oil, wherein: 2 i) wherein the weight ratio of total amount of glycerol and triglyceride oil to the amount of niacinamide is 2:1 or greater; 3 ii) wherein the weight ratio of glycerol to niacinamide is 1 :1 or greater; 4 iii) wherein the weight ratio of triglyceride oil to niacinamide is 1 :1 or greater.

The method of controlling malodour offered by the present invention is particularly useful because the benefit can extend for many hours, for example 10 hours, 24 hours, or even longer, after application of the product. This can represent an extended deodorancy benefit; that is to say, extended inhibition of the generation of odour on the human body or closely associated articles.

The method of achieving an anti-microbial and deodorancy benefit of the present invention is most efficacious when it comprises topical application of the composition directly to the human body. It is further preferred that the composition is sprayed onto the surface of the human body.

The composition of the invention may be applied to the human body by any means. Application of liquid compositions may be by absorption onto a carrier matrix like paper, fabric, or sponge and application by contacting said carrier matrix with the surface of the body. Solid or semi-solid compositions may be applied by direct contact or may be dissolved or dispersed in a liquid medium prior to application. Application may also comprise a combination of any two or more of the above techniques.

Essential ingredients of compositions of the present invention are niacinamide, glycerol and triglyceride oil.

Niacinamide is pyridine-3-carboxamide, otherwise known as nicotinamide, nicotinic acid amide and vitamin PP. It is the amide of nicotinic acid (vitamin B3/niacin).

Glycerol, also known as glycerine or glycerine, is 1,2,3-trihydroxypropane.

Herein, "triglyceride oil" is an ester of glycerol with three fatty acids, fatty acids having an even number of carbon atoms of from 4 to 28. Herein, "triglyceride oil" is sometimes abbreviated to "triglyceride." In all combinations and preferences for triglyceride oil, sunflower oil may be considered the triglyceride oil in preferred compositions.

Herein, all amounts, percentages and ratios are by weight, unless otherwise indicated. In addition, all percentages are by weight of the total composition. Herein, all amounts, percentages and ratios are to be understood as prefixed by the word "about".

Herein, any material, ratio or weight indicated as "preferred" is to be understood as preferably used in combination within any other material, ratio or weight indicated as "preferred".

Herein, the word "comprising" and "comprised of", etc., should be understood as meaning that other components/features could also be present; i.e. the listed steps or options need not be exhaustive.

The weight ratio of total amount of glycerol and triglyceride to the amount of niacinamide is 2:1 or greater.

The niacinamide is employed at a total level of from 0.5 to 10%, more preferably at from 1 to 8% and most preferably at from 2 to 5%.

The glycerol is preferably employed at a total level of from 1 to 10%, more preferably at from 2 to 8% and most preferably at from 3 to 5%.

The weight ratio of glycerol to niacinamide is 1:1 or greater. The triglyceride is preferably employed at a total level of from 1 to 10%, more preferably at from 2 to 8% and most preferably at from 3 to 5%.

The weight ratio of triglyceride to niacinamide is 1:1 or greater.

The weight ratio of glycerol to triglyceride is preferably from 1:3 to 3:1, more preferably from 1:2 to 2:1 and most preferably from 2:3 to 3:2.

In order to gain the desired level of performance, the total amount of glycerol and triglyceride is greater than the amount of niacinamide present in the composition. The weight ratio of the total amount of glycerol and triglyceride to the amount of niacinamide is 2:1 or greater. The reason for the performance enhancement delivered by the glycerol and triglyceride is not certain, but it may relate to enhanced spreading or penetration of the niacinamide into the skin (or the pores therein) or enhanced penetration through bacterial cells walls, thereby reducing bacterial numbers more effectively.

The triglyceride oil is unsaturated, i.e. it preferably comprises one or more unsaturated fatty acid residues. The preferred chain length for the fatty acids is from 12 to 18 carbon atoms. Particularly preferred triglyceride oils have fatty acids selected from oleic acid, linoleic acid or recinoleic acid.

Natural triglyceride oils are preferred, in particular coriander seed oil, borage seed oil, evening primrose oil, maize oil, sunflower seed oil and safflower seed oil. Sunflower seed oil is particularly preferred.

Water is an essential component of compositions of the invention and may function as a liquid carrier fluid. Other liquid components may also be present, including both hydrophilic and hydrophobic materials.

For many applications, it is desired that more than 50% by weight of water is present as part of the composition, more preferably more than 60%. For some preferred compositions, the ratio of other liquid components to water is between 5:95 and 15:85, by weight.

Preferred compositions are oil-in-water emulsion compositions stabilised by nonionic emulsifier. Preferred emulsifiers for this purpose form a lamellar phase. Mixtures of nonionic surfactants are preferred for this purpose, for example stearyl alcohol ethoxylated with an average of two EO units in admixture with stearyl alcohol ethoxylated with an average of 20 or 21 EO unit.

Preferred compositions are suitable for use as roll-on compositions, i.e. they are suitable for application using a roll-on dispenser.

### Optional Additional Components

A fragrance is a highly preferred additional component in compositions according to the present invention. The presence of a fragrance may enhance the deodorancy benefit delivered, whether by a masking effect or otherwise and may lead to a synergistic enhancement of the deodorancy benefit.

Herein, the terms "fragrance" and "perfume" are used interchangeably and can refer to single fragrance or perfume accords or multiple such accords.

When a fragrance is used, it may be in the form of a free (non-encapsulated) fragrance, typically emulsified, or it may be encapsulated in one of the multiple encapsulating materials used for this purpose. Encapsulated fragrances are a preferred feature of compositions of the invention, especially in compositions also comprising a non-encapsulated fragrance. Compositions of the invention including both encapsulated and non-encapsulated fragrance can deliver enhanced, long-lasting deodorancy.

Fragrance may be advantageously employed at a total level of from 0.1 to 6%, preferably 0.5 to 5% and more preferably at from 1 to 4% by weight of the total composition, excluding any volatile propellant that may be present therein.

Other liquid components that may be sued include silicone oils, such as cyclic or linear silicones, examples including Dow Corning silicone fluids 344, 345, 244, 245, 246, 556, and the 200 series; Union Carbide Corporation Silicones 7207 and 7158; and General Electric silicone SF1202. Alternatively, or additionally, non-silicone hydrophobic liquids may be used. Such materials include mineral oils, hydrogenated polyisobutene, polydecene, paraffins, isoparaffins of at least 10 carbon atoms, aliphatic or aromatic ester oils (eg. isopropyl myristate, lauryl myristate, isopropyl palmitate, diisopropyl sebecate, diisopropyl adipate, or C₈ to C18 alkyl benzoates), and polyglycol ethers, for example polyglycol butanol ethers.

Inorganic antimicrobial agents, in particular antiperspirant salts that are aluminium, zirconium and aluminium/zirconium halides and halohydrate salts, are preferably absent from compositions of the present invention. Hence, preferred compositions exclude any aluminium-containing antiperspirant active. The present inventors have found that acceptable deodorancy benefits can be achieved without the use of such astringent materials.

The ethanol content of compositions of the invention is preferably kept to a minimum, by which is meant the level is kept below 1%, preferably below 0.5% and more preferably below 0.1%. Most preferably, ethanol is completely absent from compositions of the invention.

In particularly preferred embodiments, both ethanol and inorganic antimicrobial agents, in particular antiperspirant salts that are aluminium, zirconium and aluminium/zirconium halides and halohydrate salts, are absent.

An organic antimicrobial agent other than ethanol or niacinamide is a preferred additional component of compositions of the invention. Particular preferred organic antimicrobials are selected from iodopropynyl butylcarbamate, phenoxyethanol and pentaerythrityl tetra-di-t-butyl hydroxyhydrocinnamate. This optional component can expand the range of microbes against which the composition is active. Organic antimicrobials other than ethanol or niacinamide are preferably used a total level of from 0.1 to 1%.

Structurants and emulsifiers are further additional components of the compositions of the invention that are highly desirable in certain product forms. Structurants, when employed, are preferably present at from 1% to 30% by weight of the composition, whilst emulsifiers are preferably present at from 0.1% to 10% by weight of the composition. Suitable structurants include cellulosic thickeners such as hydroxy propyl cellulose and hydroxy ethyl cellulose, and dibenzylidene sorbitol. Other suitable structurants include sodium stearate, stearyl alcohol, cetyl alcohol, hydrogenated castor oil, synthetic waxes, paraffin waxes, hydroxystearic acid, dibutyl lauroyl glutamide, alkyl silicone waxes, and silica. Suitable emulsifiers include steareth-2, steareth-20, steareth-21, ceteareth-20, glyceryl stearate, cetyl alcohol, cetearyl alcohol, PEG-20 stearate, dimethicone copolyol, and poloxamines.

Further emulsifiers/surfactants desirable in certain compositions of the invention are perfume solubilisers and wash-off agents. Examples of the former include PEG-hydrogenated castor oil, available from BASF in the Cremaphor RH and CO ranges, preferably present at up to 1.5% by weight, more preferably 0.3 to 0.7% by weight. Examples of the latter include poly(oxyethylene) ethers.

Certain sensory modifiers are further desirable components in the compositions of the invention. Such materials are preferably used at a level of up to 20% by weight of the composition. Emollients, humectants, volatile oils, non-volatile oils, and particulate solids which impart lubricity are all suitable classes of sensory modifiers. Examples of such materials include cyclomethicone, dimethicone, dimethiconol, isopropyl myristate, isopropyl palmitate, polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

### Examples

The roll-on compositions detailed in Table 1 were prepared by methods known in the art.

The deodorancy performance of the compositions was compared in a head-to-head test as detailed below.

A panel of 50 female panellists was employed. At the start of the test, panellists were washed with unfragranced soap and different treatments applied to each axilla using a conventional roll-on applicator with a target dose of 0.30g (+/- 30mg) via strokes of the roll-on. Application was randomised to take into account any left/right bias. Panellists were instructed not to consume spicy food or alcohol, and not to wash under their own axillae, during the duration of the test.

**Table 1**

| **Component** | | **Example 1** | **Control** |
|---|---|---|---|
| **RM name** | **Hierarchy name** | **Wt %** | |
| Niacinamide | Niacinamide | 5.00 | -- |
| Sunflower seed oil | *Helianthus anuus* seed oil | 4.00 | 4.00 |
| Glycerin | Glycerol | 4.00 | 4.00 |
| Steareth-2 | Ethoxylated alcohol C18/E2 | 2.60 | 2.60 |
| Steareth-20 | Ethoxylated alcohol C18/E20 | 0.60 | 0.60 |
| Phenoxyethanol | 2-phenoxyethanol | 0.40 | 0.40 |
| Glycacil L* | 3-iodo-2-propynyl butyl carbamate, PEG laurate and PEG dilaurate | 0.07 | 0.07 |
| Water | Water | To 100 | To 100 |
| **MMS** (mean malodour score) | After 5 hours: | 2.07 | 2.19 |
| | After 24 hours: | 2.49 | 2.61 |

| | | | |
|---|---|---|---|
| * Glycacil L comprises 10% of the active ingredient (3-iodo-2-propynyl butyl carbamate). | | | |

At least three expert assessors determined the intensity of axillary odour after 5 hours and after 24 hours, scoring the intensity on a scale of 0-5 (where 0 = no odour). Three further repeat cycles were performed. At the end of the test, the data were analysed using standard statistical techniques.

It was found that treatment with Example 1 led to a significant lower mean malodour score after both 5 and 24 hours (significant at >99%).

The roll-on compositions detailed in Table 2 are examples of further compositions according to the invention and may be prepared by methods known in the art. The components sharing RM names with those in Table 1 are the same raw materials as used in the Table 1 examples.

**Table 2**

| **Component** | **Example 2** | **Example 3** | **Example 4** | **Example 5** |
|---|---|---|---|---|
| **RM name** | **Wt %** | | | |
| Niacinamide | 3.00 | 2.00 | 4.00 | 3.00 |
| Sunflower seed oil | -- | -- | 5.00 | 4.00 |
| Coriander seed oil | 3.00 | -- | -- | |
| Maize oil | -- | 4.00 | -- | -- |
| Glycerin | 5.00 | 2.00 | 4.00 | 3.00 |
| Steareth-2 | 2.60 | 2.60 | 2.60 | 2.60 |
| Steareth-20 | | 0.60 | 0.60 | 0.60 |
| Phenoxyethanol | 0.40 | 0.20 | 0.40 | -- |
| Pentaerythrityl tetra-dit-butyl hydroxyhydrocinnamate | -- | 0.15 | 0.20 | 0.10 |
| Water | To 100 | To 100 | To 100 | To 100 |

## Claims

1. A non-therapeutic method of reducing malodour on the surface of the human body, comprising the topical application of a deodorant composition comprising water, niacinamide at from 0.5 to 10% by weight, glycerol and an unsaturated triglyceride oil, wherein:
(i) the weight ratio of total amount of glycerol and triglyceride oil to the amount of niacinamide is 2:1 or greater;
(ii) the weight ratio of glycerol to niacinamide is 1:1 or greater and
(iii) the weight ratio of triglyceride oil to niacinamide is 1:1 or greater.

2. A method according to claim 1, wherein the triglyceride oil is sunflower seed oil.

3. A method according to any of the preceding claims, wherein the water content is greater than 50% by weight.

4. A method according to any of the preceding claims, wherein the composition is an oil-in-water emulsion stabilised by nonionic surfactant.

5. A method according to any of the preceding claims, wherein the deodorant composition comprises an encapsulated fragrance.

6. A method according to any of the preceding claims, wherein the deodorant composition excludes ethanol and any aluminium-containing antiperspirant active.

7. A method according to any of the preceding claims, wherein the deodorant composition is applied by a roll-on dispenser directly onto the surface of the human body.

## Patentansprüche

1. Nicht-therapeutisches Verfahren zur Reduzierung von unangenehmem Geruch auf der Oberfläche des menschlichen Körpers, umfassend die topische Anwendung einer Deodorantzusammensetzung, umfassend Wasser, Niacinamid mit 0,5 bis 10 Gewichts-%, Glycerol und ein ungesättigtes Triglyceridöl, wobei:
(i) das Gewichtsverhältnis der Gesamtmenge des Glycerols und Triglyceridöls zu der Menge des Niacinamids 2:1 oder mehr beträgt;
(ii) das Gewichtsverhältnis von Glycerol zu Niacinamid 1:1 oder mehr beträgt und
(iii) das Gewichtsverhältnis von Triglyceridöl zu Niacinamid 1:1 oder mehr beträgt.

2. Verfahren nach Anspruch 1, wobei das Triglyceridöl Sonnenblumenöl ist.

3. Verfahren nach irgendeinem der vorhergehenden Ansprüche, wobei der Wassergehalt mehr als 50 Gewichts-% beträgt.

4. Verfahren nach irgendeinem der vorhergehenden Ansprüche, wobei die Zusammensetzung eine ÖI-in-Wasser-Emulsion ist, stabilisiert durch nicht-ionisches Tensid.

5. Verfahren nach irgendeinem der vorhergehenden Ansprüche, wobei die Deodorantzusammensetzung einen eingekapselten Duftstoff umfasst.

6. Verfahren nach irgendeinem der vorhergehenden Ansprüche, wobei die Deodorantzusammensetzung Ethanol und irgendeinen Aluminium-enthaltenden schweißhemmenden Wirkstoff ausschließt.

7. Verfahren nach irgendeinem der vorhergehenden Ansprüche, wobei die Deodorantzusammensetzung mittels eines Roll-on-Spenders direkt auf die Oberfläche des menschlichen Körpers aufgetragen wird.

## Revendications

1. Procédé non-thérapeutique de réduction de mauvaises odeurs sur la surface du corps humain, comprenant l'application topique d'une composition de déodorant comprenant de l'eau, du niacinamide à de 0,5 à 10 % en masse, du glycérol et une huile de triglycéride insaturée, dans lequel :
(i) le rapport de masse de quantité totale de glycérol et d'huile de triglycéride à la quantité de niacinamide est de 2:1 ou supérieur ;
(ii) le rapport de masse de glycérol à niacinamide est de 1:1 ou supérieur et
(iii) le rapport de masse d'huile de triglycéride à niacinamide est de 1:1 ou supérieur.

2. Procédé selon la revendication 1, dans lequel l'huile de triglycéride est l'huile de graine de tournesol.

3. Procédé selon l'une quelconque des revendications précédentes, dans lequel la teneur en eau est supérieure à 50 % en masse.

4. Procédé selon l'une quelconque des revendications précédentes, dans lequel la composition est une émulsion huile-dans-eau stabilisée par un tensioactif non ionique.

5. Procédé selon l'une quelconque des revendications précédentes, dans lequel la composition de déodorant comprend un parfum encapsulé.

6. Procédé selon l'une quelconque des revendications précédentes, dans lequel la composition de déodorant exclut l'éthanol et tout actif antiperspirant contenant de l'aluminium.

7. Procédé selon l'une quelconque des revendications précédentes, dans lequel la composition de déodorant est appliquée par un distributeur à bille directement sur la surface du corps humain.
